Europäisches Patentamt

European Patent Office

Office européen des brevets

(19)

(11) Numéro de publication: **0 065 895**
**B1**

(12) ## FASCICULE DE BREVET EUROPEEN

(45) Date de publication du fascicule du brevet:
19.09.84

(51) Int. Cl.³: **C 12 N 1/00**, C 12 N 1/20,
C 12 M 1/12

(21) Numéro de dépôt: 82400775.1

(22) Date de dépôt: 29.04.82

(54) Procédé de fabrication de microorganismes.

(30) Priorité: 08.05.81 FR 8109153

(43) Date de publication de la demande:
01.12.82 Bulletin 82/48

(45) Mention de la délivrance du brevet:
19.09.84 Bulletin 84/38

(84) Etats contractants désignés:
AT BE CH DE FR GB IT LI LU NL SE

(56) Documents cités:
FR - A - 2 238 759
US - A - 3 418 208
US - A - 3 472 765
US - A - 4 167 450

APPLIED AND ENVIRONMENTAL MICROBIOLOGY, vol. 34, no. 6, décembre 1977, pages 733-739, Washington (USA);
APPLIED AND ENVIRONMENTAL MICROBIOLOGY, vol. 34, no. 6, décembre 1977, pages 725-732, Washington (USA);
APPLIED AND ENVIRONMENTAL MICROBIOLOGY, vol. 37, no. 3, mars 1979, pages 487-495, Washington DC. (USA);
JOURNAL OF DAIRY SCIENCE, vol. 63, no. 5, 1980, pages 722-730, Champaign Ill. (USA);

(73) Titulaire: **L'AIR LIQUIDE, SOCIETE ANONYME POUR L'ETUDE ET L'EXPLOITATION DES PROCEDES GEORGES CLAUDE, 75, Quai d'Orsay, F-75321 Paris Cedex 07 (FR)**

(72) Inventeur: **Amen, Jean, 108, bd de la Reine, F-78000 Versailles (FR)**
Inventeur: **Cabau, Michel, Le Plan du Guillou, F-38500 Coublevie Voiron (FR)**

(74) Mandataire: **Leclercq, Maurice et al, L'AIR LIQUIDE SOCIETE ANONYME POUR L'ETUDE ET L'EXPLOITATION DES PROCEDES GEORGES CLAUDE 75, Quai d'Orsay, F-75321 Paris Cedex 07 (FR)**

## Description

La fabrication de microorganismes s'effectue dans un milieu de culture incorporant des substrats nutritifs, dilué dans de l'eau. Ce milieu de culture est ensemencé initialement en microorganismes qui se développent ainsi en milieu quasi fermé, sauf l'apport d'agent neutralisant (culture dite en »batch«). Ce type de fermentation, utilisé à échelle industrielle présente l'avantage de la simplicité, mais une faible efficacité, car il existe des facteurs limitants, soit dans le substrat nutritif lui-même, soit dans les agents inhibiteurs produits par les microorganismes, et dont la concentration de plus en plus élevée au cours de la fermentation a pour effet de réduire le taux de croissance des microorganismes.

Pour pallier cet inconvénient, on a proposé d'assurer une fermentation en continu, c'est-à-dire d'assurer à la fois une extraction dosée du milieu de culture simultanément à une alimentation également dosée en substrat nutritif, neutralisant et eau. Cette façon de faire permet d'améliorer, pour un fermenteur de capacité volumique donnée, la production des microorganismes, mais cela au prix d'une concentration réduite des microorganismes dans l'extrait que l'on opère sur le milieu de culture, donc avec en fait un rendement de microorganismes-substrat nutritif assez faible.

Une autre façon de faire est de ménager ce même milieu de culture dans un fermenteur de taille nettement supérieur au volume initial dudit milieu de culture et d'ajouter de façon continue le substrat nutritif dilué dans de l'eau et l'agent neutralisant. Ce type de fermentation (dit »feed batch«), qui permet de réduire l'effet néfaste des inhibiteurs en maintenant leur concentration à un niveau limité, présente cependant l'inconvénient majeur de nécessiter impérativement un fermenteur de capacité très surdimensionné, en sorte que cette solution n'a que très rarement été retenue par l'industrie.

On a également proposé d'utiliser des membranes de dialyse qui permettent, dans certaines conditions, d'éliminer les agents inhibiteurs formés et ainsi d'accroître les concentrations finales en microorganismes du milieu de culture, mais cette solution n'a pas été retenue par l'industrie parce que de telles membranes sont nettement trop fragiles, à la fois mécanique ment, thermiquement et chimiquement, parce que, également, leur maîtrise opératoire est délicate et complexe, leur mode opératoire à volume variable peu sohaitable et le rendement en poids de microorganismes produits relativement au substrat introduit dans le milieu reste faible.

La technique des membranes d'ultrafiltration a permis de franchir un pas en avant dans la fermentation de produits biologiques et cela par passage forcé d'un débit recyclé dudit milieu de culture dans une cellule d'ultrafiltration, dont on peut choisir les caractéristiques de façon à assurer l'extraction du et des agents inhibiteurs, soutirés ainsi du milieu de culture avec, cependant et inévitablement, une partie de substrats nutritifs, alors que les microorganismes sont maintenus dans le débit recyclé dans le milieu de culture. On parvient ainsi à accroître la concentration finale en microorganismes dans le milieu de culture, et cela dans des proportions de l'ordre de deux à trois par rapport au procédé dit en »batch«. Cependant, on constate dans ce dernier procédé, même avec ses remarquables qualités, une réduction très nette du taux de croissance des microorganismes après un développement initial élevé. Le but, à l'origine de la présente invention, a été de rechercher les contitions opératoires qui permettraient si possible d'accroître encore, dans un procédé de ce type, la concentration finale en microorganismes tout en conservant un rendement acceptable en poids relatif de la consommation des substrats nutritifs.

Ce résultat est atteint, selon l'invention, dans un procédé du type où l'on effectue sur le milieu de culture une opération d'ultrafiltration d'élimination partielle d'un agent inhibiteur formé et cela pendant au moins une partie de la phase de développement des dits microorganismes, du genre où l'on assure un taux de croissance des microorganismes dudit milieu de culture qui présente, dans une première phase initiale de développement, une valeur élevée et dans une phase successive une valeur constante plus modérée, par la combinaison des mesures suivantes:

a) pendant toute la durée de la phase de fabrication, on régule l'apport en agent neutralisant pour maintenir le PH du milieu de culture à une valeur strictement constante, ne dépassant pas 7,5 et comprise entre 5,5 et 7,5;

b) on règle les apports en substrat et en eau par rapport à l'apport en agent neutralisant et cela, par détermination de la concentration en agent inhibiteur résultant de l'action dudit agent neutralisant;

c) l'ensemble de ces apports s'effectue directement dans le fermenteur durant toute la fabrication;

d) le taux de croissance modéré est compris entre 0,10 et 0,50;

e) le début de la phase à taux de croissance modéré est déterminé par l'obtention d'une concentration critique dans le milieu de culture de l'agent inhibiteur, qui corresponde à une concentration maximale admissible pour ledit taux de croissance modéré choisi;

f) on asservit au moins la phase essentielle de l'opération d'ultafiltration au maintien d'un volume constant dudit milieu de culture, dans lequel se retrouve la totalité des microorganismes fabriqués.

Chacune de ces conditions concourt au résultat escompté: le réglage à valeur strictement constante du PH par un agent neutralisant a pour effet de permettre de doser très exactement les apports en substrat et en eau. Puisqu'on fait appel à un maintien précis du PH, on peut très simplement piloter le déroulement de la fermentation par la connaissance de la concentration en agent inhibiteur et, puisqu'il existe une corrélation entre cette concentration en agent inhibiteur et le taux de croissance, on peut ainsi très aisément, par ce moyen, modifier, après une phase initiale, les conditions d'alimentation en eau, et éventuellement en substrat, pour assurer un taux de croissance quelque peu plus modéré, qui pourra être maintenu, tout au long du processus de fermentation, tandis que la capacité d'ultrafiltration à mettre en oeuvre pour maintenir un volume constant est elle-même étroitement dépendante du taux de croissance retenu. En effet, on comprend qu'à taux de croissance plus élevé correspond une concentration en agent inhibiteur plus faible, donc à une plus grande capacité d'ultrafiltration. C'est un des mérites de l'invention de proposer, pour la seconde phase de développement, des taux de croissance qui peuvent s'étendre de 0,10 à 0,50 mais qui, de façon avantageuse, sont compris entre 0,15 et 0,45, et encore plus avantageuse entre 0,20 et 0,40. On a pu même établir, pour certaines applications, que la valeur optimale du taux de croissance plus modéré se situe entre 0,30 et 0,35. Ainsi qu'il a été précisé, à chaque taux de croissance retenu et pour chaque microorganisme cultivé correspond une valeur maximale critique de la concentration en agent inhibiteur et c'est précisément lorsque cette valeur maximale critique est atteinte que l'on procède aux adaptations à la phase successive de développement à taux de croissance plus modéré.

Selon une particularité essentielle de mise en oeuvre du procédé selon l'invention, les débits de substrats et d'eau sont maintenus à des rapports constants relativement au débit d'agent neutralisant pendant toute la phase de développement initial à taux de croissance élevé et, dans d'autres rapports, pendant toute la phase successive de développement à taux de croissance plus modéré, pendant laquelle le débit relatif d'eau est rendu plus élevé, d'autant plus élevé que la concentration maximale admissible en agent inhibiteur est choisie plus faible. Cette façon de faire qui consiste à faire dépendre par une liaison opérationnelle constante et optimalisée les débits d'alimentation a pour résultat une dépense minimale en substrat et une adéquation aussi parfaite que possible des besoins nutritifs des microorganismes à la croisssance que l'on décide de leur imprimer. Bien que les adaptations de la phase initiale à la phase successive puisse paraître de faible envergure, sans de telles adaptations, le succès relatif au rendement en poids substrat-microorganismes ne pourrait être atteint.

L'invention vise, entre autres, l'application à la fabrication de bactéries lactiques, du genre où l'on alimente un milieu de culture, préalablement ensemencé, en substrat nutritif comportant avantageusement du lactose et de l'extrait de levure, et en ammoniac (ou soude) formant agent neutralisant, le tout dilué dans de l'eau et où l'on effectue sur ledit milieu de culture une opération d'éliminnation partielle de lactate d'ammonium (ou de sodium) et cela pendant au moins une partie de la phase de développement des dites bactéries lactiques, du genre où l'on assure un taux de croissance des bactéries dudit milieu de cultlure qui présente, dans une première phase initiale de développement, une valeur é levée et dans une phase successive une valeur constante plus modérée, et dans cette application, l'invention prévoit la combinaison des mesures opératoires suivantes:

a) pendant toute la durée de fabrication, on régule l'apport en ammoniac (ou soude) pour maintenir le PH du milieu de culture à une valeur strictement constante, comprise entre 6 et 7;

b) on règle les apports en substrat et en eau par rapport à l'apport préréglé selon a) en ammoniac (ou soude), et cela par détermination de la concentration en lactate d'ammonium (ou sodium) résultant de l'action de l'ammoniac (ou soude) sur l'acide lactique formé lors de la dégradation du lactose;

c) le début de la phase à taux de croissance plus modéré est déterminé par l'obtention d'une concentration critique, dans le milieu de culture, du lactate d'ammonium (ou sodium), la concentration critique en lactate d'ammonium étant comprise entre 19 g/l pour un taux de croissance de 0,50 et 54 g/l pour un taux de croissance de 0,10;

d) on asservit au moins la phase essentielle de l'opération d'ultrafiltration au maintien d'un volume constant dudit milieu de culture.

Comme on le constate, il s'agit ici d'une adaptation du procédé précédemment décrit à une fabrication selon des modalités particulières exclusivement destinée aux bactéries lactiques. Comme précédemment, ce qui est décisif, c'est la détermination préalable du taux des croissance de la seconde phase de développement (permettant d'obtenir une fermentation complète dans un laps de temps déterminé) et à ce taux de croissance correspond une concentration maximale critique en lactate d'ammonium (ou sodium) qui, d'ailleurs est différente d'une bactérie à l'autre. Ainsi, on a pu établir en faisant des tests sur un très grand nombre de souches que les concentrations critiques en lactate d'ammonium s'établissent entre environ 19 g/l (taux de croissance 0,50) et environ 54 g/l (taux de croisssance 0,10) et plus généralement entre 25 g/l et 35 g/l. Une approche essentielle du procédé selon l'invention est donc la détermination préalable de la concentration critique en agent inhibiteur associé au taux de croissance retenu.

L'invention a également pour objet une installation de fabrication de microorganismes qui sera détaillée ultérieurement.

Les caractéristiques et avantages de l'invention ressortiront d'ailleurs de la description qui suit, en référence aux dessins annexés parmi lesquels:

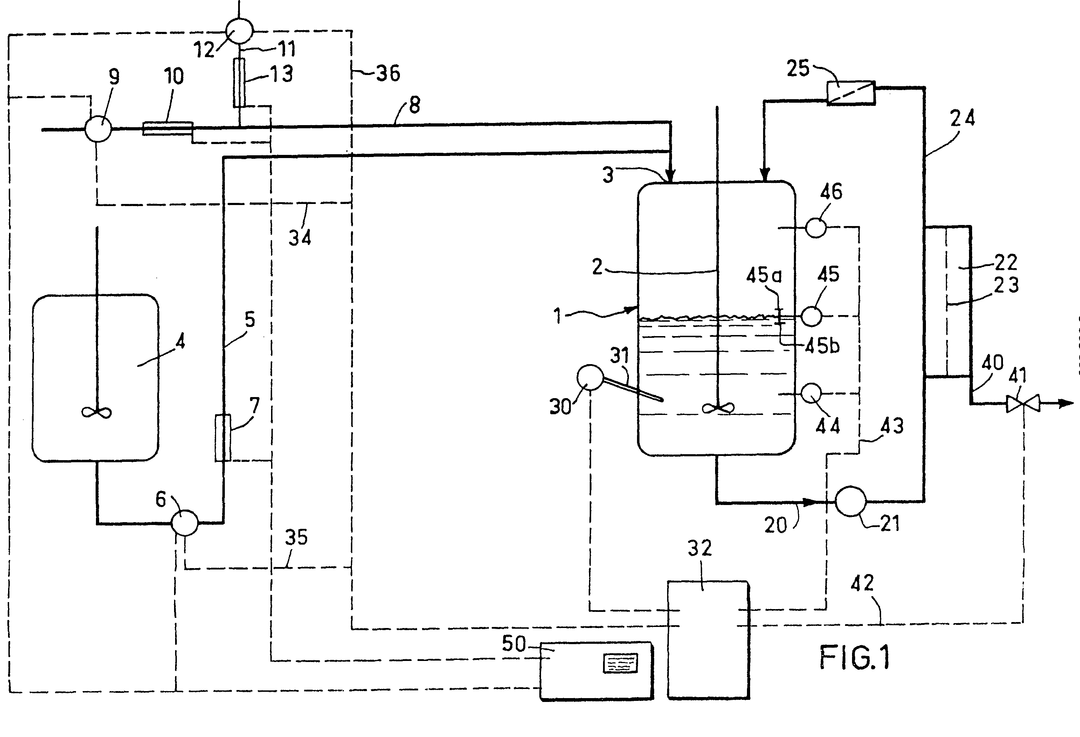

— la figure 1 est une vue schématique d'une installation de fabrication de bactéries lactiques selon l'invention;

— les figures 2 à 5 sont des diagrammes de détermination du taux de croissance.

En se référant aux dessins, un fermenteur 1 équipé d'un moyen de brassage 2 est alimenté à son extrémité supérieure 3 d'une part en substrat nutritif (lactose et levure) provenant d'un réservoir 4 au moyen d'une conduite 5 équipée d'une pompe 6 et d'un débimètre-totaliseur 7, d'autre part en ammoniaque de neutralisation par une conduite 8 équipée d'une pompe 9 et d'un débimètre-totaliseur 10, enfin en eau stérile par une conduite 11 équipée d'une pompe 12 et d'un débimètre-totaliseur 13. Le fermenteur est également équipé d'une conduite de soutirage 20, incorporant une pompe 21, aboutissant à une cellule d'ultrafiltration 22 à membrane 23. La sortie, côté amont de la membrane 23, de la cellule d'ultrafiltration 22 est raccordée par une conduite 24 incorporant un échangeur de chaleur 25 au fermenteur 1. Un appareil 30 de mesure de PH a une sonde 31 engagée dans une partie basse du fermenteur 1 et transmet un signal de mesure de PH à un régulateur 32. A partir de cette information, le régulateur 32 assure essentiellement via une ligne de commande 34 le réglage approprié du débit de la pompe d'ammoniaque 9, ou de son temps de fonctionnement, de façon à maintenir le PH à une valeur constante fixée de façon prédéterminée et qui peut être réglé en fonction du type de bactérie en cours de fabrication. Le régulateur 32 agit également par une ligne de commande 35 sur le débit ou la durée de fonctionnement de la pompe de substrat 6 et, via une ligne de commande 36, sur le débit ou la durée de fonctionnement de la pompe d'eau de dilution 12.

La cellule d'ultrafiltration 22 présente de façon habituelle une sortie côté filtrat raccordée à l'évacuation par une conduite 40 incorporant une vanne de réglage 41 qui, elle-même, est asservie via une ligne de commande 42 au régulateur 32, qui reçoit à cet effet des informations, via une ligne de mesure 43, d'indicateurs de niveau minimal 44, moyen 45 et maximal 46. En fait, l'indicateur de niveau moyen 45 présente deux sondes distinctes 45a, 45b, de façon à pouvoir assurer le maintien d'un niveau constant ainsi qu'il sera détaillé dans ce qui suit:

Le fonctionnement de l'installation qui vient d'être décrite est le suivant:

Au début d'une opération de fabrication de microorganismes, on introduit dans le fermenteur 1 un volume de substrat de façon à ce que celui-ci atteigne à près le niveau de l'indicateur de niveau 45. On ensemence ce milieu de culture avec une bactérie lactique, on assure par des moyens appropriés une température de l'ordre de 30° C du milieu de culture et l'on maintient une atmosphère surplombant le milieu de culture avec de l'azote à 0,5 bar, et on met en oeuvre le moyen de brassage 2. Du substrat nutritif incorporant essentiellement, dans le réservoir 4, du lactose à une concentration $(L_0)$ et de la levure à une concentration $(E_0)$ est délivrée par la pompe 6 dans la canalisation 5 à un débit $(D_1)$ (totalisation $T_1$), tandis que de l'ammoniaque à une concentration $(N_0)$ est délivrée dans la canalisation 8 à un débit $(D_2)$ (totalisation $T_2$) tandis que de l'eau stérile est délivrée dans la canalisation 11 à un débit $(D_3)$ (totalisation $T_3$).

Pendant cette première phase, d'une part la concentration de substrat dans le fermenteur a été choisie à une valeur relativement élevée (lactose 45 g/l, extrait de levure 15 g/l) et pendant une première phase initiale de développement les concentrations en lactone (LI), levure (EI) et ammoniaque (NI) pénétrant à l'extrémité (3) dans le fermenteur 1 sont maintenus par exemple à 40 g/l pour (LI), 13,3 g/l pour (EI) et 12 N pour (NI). On notera que ces concentrations peuvent être aisément déterminées à partir du pupitre de contrôle (50), qui recueille les indications des débimètres-totaliseurs 7, 10 et 13.

Pendant toute cette phase opératoire, il importe, pour un bon déroulement du procédé, de contrôler régulièrement le taux de croissance qui doit être aussi élevé que possible.

Ce taux de croissance dans la phase exponentielle où l'on se situe pendant toute la durée du procédé de fabrication est représenté par la constante d'accroîssement logarithmique de la masse biologique en cours de développement en fonction du temps, selon la formule $X = X_0 e^{\mu t}$ (où $\mu$ est le taux de croissance) et l'on démontre d'ailleurs, dans le cas de l'exemple choisi, que le lactate d'ammonium qui se forme par la combinaison de l'acide lactique (résultant de la consommation de lactose) et de l'ammoniaque introduit est relié aux taux de croissance par la relation:

$$Lg_n \frac{dL}{dt} = \mu \cdot t + const.,$$

et l'on note que, la détermination de la concentration en lactate d'ammonium étant directement proportionnelle à l'admission d'ammoniaque (avec un milieu de culture à PH constant), il suffit de noter la variation du débit d'ammoniaque pour déterminer le taux de croissance en fonction du temps:

$$\mu = Lg \frac{dL}{dt} \Big/ t.$$

On conçoit donc qu'on peut suivre à chaque instant et, le cas échéant, par la voie de tracé graphique

$$Lg_n \frac{dL}{dt} = \mu t + const.,$$

la variation éventuelle du taux de croissance, représenté par la pente de la courbe ainsi tracée, et que par des corrections appropriées, on peut au contraire maintenir constant ce taux de croissance à une valeur prédéterminée par un réglage correctif des apports en substrat et en eau par rapport à l'apport de neutralisant.

Pendant toute une première phase opérationnelle, la pompe 21 du circuit d'ultrafiltration est hors de fonctionnement et le niveau du milieu de culture s'élève progressivement depuis le niveau indiqué par l'indicateur 45 jusqu'au niveau indiqué par l'indicateur 46 (volume maximal). Ce n'est que lorsque ce niveau 46 est atteint que le régulateur 32 met en oeuvre, via la ligne de commande 42, l'ouverture régulée de la vanne 41, ce qui permet à l'ultrafiltrat d'être évacué et de commencer réellement l'opération d'ultrafiltration.

Ainsi, de façon connue en soi, les agents inhibiteurs tels que le lactate d'ammonium sont éliminés à travers la membrane d'ultrafiltration 23, alors qu'au contraire les bactéries lactiques sont recyclées via la canalisation 24 vers le fermenteur 1. Le niveau du milieu de culture est réduit rapidement jusqu'à atteindre le niveau intermédiaire contrôlé par l'indicateur 45 et une fois ce niveau atteint, le régulateur 32 est programmé pour que la vanne 41 se ferme quelque peu de façon à assurer un niveau quasi constant grâce aux deux sondes de niveau intermédiaire établi à faible distance 45a et 45b.

Un contrôle des opérations, pendent toute cette période, est assuré, notamment du taux de croissance qui, comme on l'a vu, est déterminé par les variations logarithmiques dans le temps de la concentration du lactate d'ammonium dans le fermenteur. Une fois qu'une concentration maximale en lactate, qui a été initialement prédéterminée, est atteinte, qui correspond pour la bactérie cultivée à un taux de croissance donné, on fait en sorte que cette concentration en lactate d'ammonium soit maintenue constante, ce qui a donc pour effet de maintenir constant le taux de croissance des bactéries. A cet effet, dès que cette valeur maximale de la concentration en lactate d'ammonium est atteinte, on augmente quelque peu le débit d'eau fournie par la pompe 12, de façon à introduire dans le fermenteur des quantités d'ammoniaque qui correspondent très exactement au maintien constant de la concentration en lactate d'ammonium. On peut aisément déterminer le débit ($D_3$) de la pompe 12 pour que la concentration en ammoniaque introduit soit celle qui correspond à la concentration, dans le fermenteur, en lactate d'ammonium, ce qui assure ainsi son maintien.

On peut ainsi être assuré de maintenir à un niveau strictement constant le taux de croissance, le cas échéant par un réglage complémentaire d'ajustement de l'apport en substrat, le tout en maintenant le PH constant. Il y a lieu de noter que la mise en oeuvre de la phase d'ultrafiltration est asservie à l'obtention par le milieu de culture du niveau repéré par l'indicateur de niveau 46, de sorte que l'intervention de l'ultrafiltration, selon le cas, peut précéder ou suivre la modification des conditions d'alimentation qui découlent de la fixation à un niveau plus modéré du taux de croissance des bactéries lactiques.

A titre d'exemple de mise en oeuvre, on donne ci-dessous, pour certaines bactéries lactiques, les concentrations critiques en lactate d'ammonium pour un taux de croissance de 0,35 avec l'indication de provenance:

CNRZ: bactéries disponibles au »Centre National de Recherches de Zootechniques« de Jouy-en-Josas (France);

NCDO: bactéries disponibles à la »National Collection of Dairy Organisms« de Reading (Angleterre) et:

(S. L.) signifiant »streptococcus lactis«
(S. C$_r$) signifiant »streptococcus cremoris«
(S. D.) signifiant »streptococcus diacetyl lactis«.

| Type de bacterie | concentration critique ($\mu = 0,35$) (lactate d'ammonium) |
|---|---|
| CNRZ 269 (SL) | 30 g/l |
| NCDO 763 (SL) | 26 g/l |
| CNRZ 29 (SL) | 38 g/l |
| NCDO 505 (SL) | 38 g/l |
| NCDO 1198 (SL) | 26 g/l |
| CNRZ 116 (SCr) | 23 g/l |
| NCDO 1009 (SCr) | 21 g/l |
| NCDO 699 (SCr) | 26 g/l |
| CNRZ 253 (SCr) | 30 g/l |
| NCDO 1000 (SCr) | 21 g/l |
| NCDO 1119 (SCr) | 28 g/l |
| NCDO 495 (SCr) | 38 g/l |
| CNRZ 124 (SD) | 28 g/l |
| CNRZ 125 (SD) | 32 g/l |

Généralement, on est parti de substrats dans le réservoir 4 aux plus fortes concentrations possibles, de l'ordre de:

(Lo) = 240 g/l (lactose)
(Eo) = 80 g/l (extrait de levure)
et d'ammoniac (No) = 12 N (Normalité) et l'on peut à chaque instant déterminer les concentrations des produits entrant dans le fermenteur 1; mais également et surtout le taux de lactate à chaque instant, avec la relation:

$$(lactate) = \frac{T_2 \cdot No \cdot 107,11}{Vo + T_1 + T_2 + T_3}$$

où

$T_1$ = volume substrat totalisé
$T_2$ = volume ammoniaque totalisé
$T_3$ = volume eau totalisé
$Vo$ = Volume initial dans le fermenteur
107,11 = Poids moléculaire du lactate d'ammonium

et le passage de la première phase initiale à taux de croissance élevé à seconde phase à taux de croissance plus modéré, s'effectue simplement en accroissant le débit d'eau $D_3$ de façon à adapter la concentration en ammoniaque entrant à la concentration en lactate d'ammonium dans le fermenteur, impliquant la détermination mathématique suivante:

$$D_3 = \frac{(No) \times 107,11 \times D_2}{(lactate)} - (D_1 + D_2)$$

et le cas échéant, à ce moment, on modifie quelque peu l'apport en substrat suivant la modération du taux de croissance.

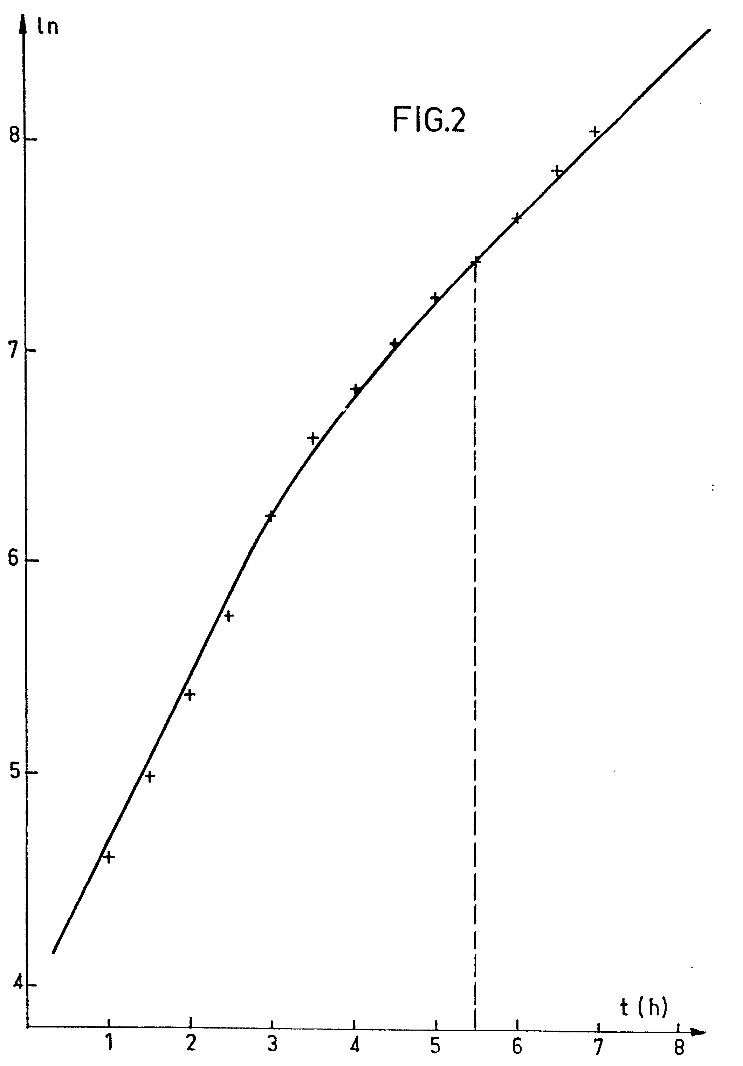

On procède maintenent à un rapide examen des figures 2 à 5 qui sont des diagrammes le taux de croissance en fonction du temps:

A la figure 2, concernant la souche CNRZ 269, on note un taux de croissance élevé de l'ordre de 0,77 pendant les trois premières heures. Après 5 H 30 mn, le taux de lactate a atteint 30 g/l, et à partir de ce moment, on a modifié l'alimentation en eau, en l'accroissant d'environ 50%, ce qui a eu pour effet de réduire la concentration en ammoniaque assurant ainsi une introduction d'am moniaque dans le fermenteur apte à maintenir cette concentration de 30 g/l qui correspond à un taux de croissance de 0,39 de cinq heures trente à huit heures. On note que l'ultrafiltration est mise en oeuvre à peu près après quatre heures. En fin de fermentation la concentration bactérienne obtenue est de 32 g de bactéries sèches par kg de culture. Le rendement en substrats est de 7,5 kg de lactose par kg de bactéries sèches.

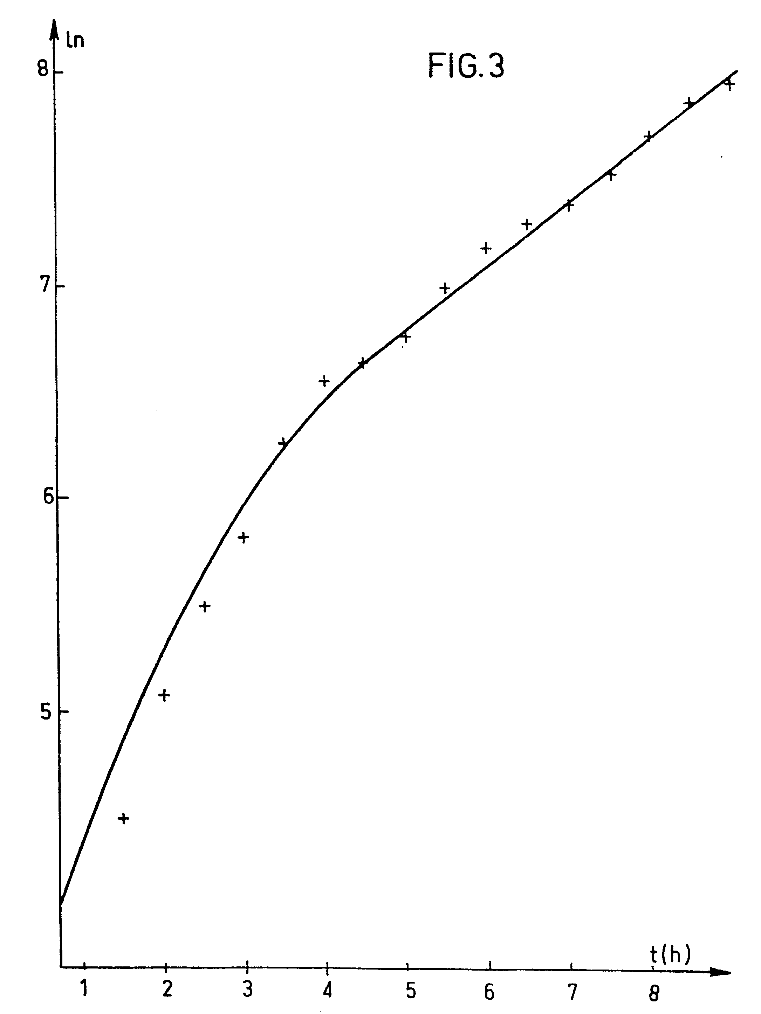

A la figure 3, concernant la souche CNRZ 116, on note un taux de croissance élevé de l'ordre de 0,71 par heure pendant les quatre premières heures. Après cinq heures, le taux de lactate a atteint 24 g/l, et à partir de ce moment, on a modifié l'alimentation en eau, en l'accroissant de 98%, ce qui a eu pour effet de réduire la concentration en ammoniaque assurant ainsi une introduction d'ammoniaque dans le fermenteur apte à maintenir cette concentration de 24 g/l qui correspond à un taux de croissance de 0,30 par heure de 5 heures à 9 heures. On note que l'ultrafiltration est mise en oeuvre un peu avant 5 heures. Le débit a été augmenté de 7% avant la

7ème heure. En fin de fermentation la concentration bactérienne est de 20 g de bactéries sèches par kilo de culture. Le rendement en substrat est de 8,4 kg de lactose par kg de bactéries sèches.

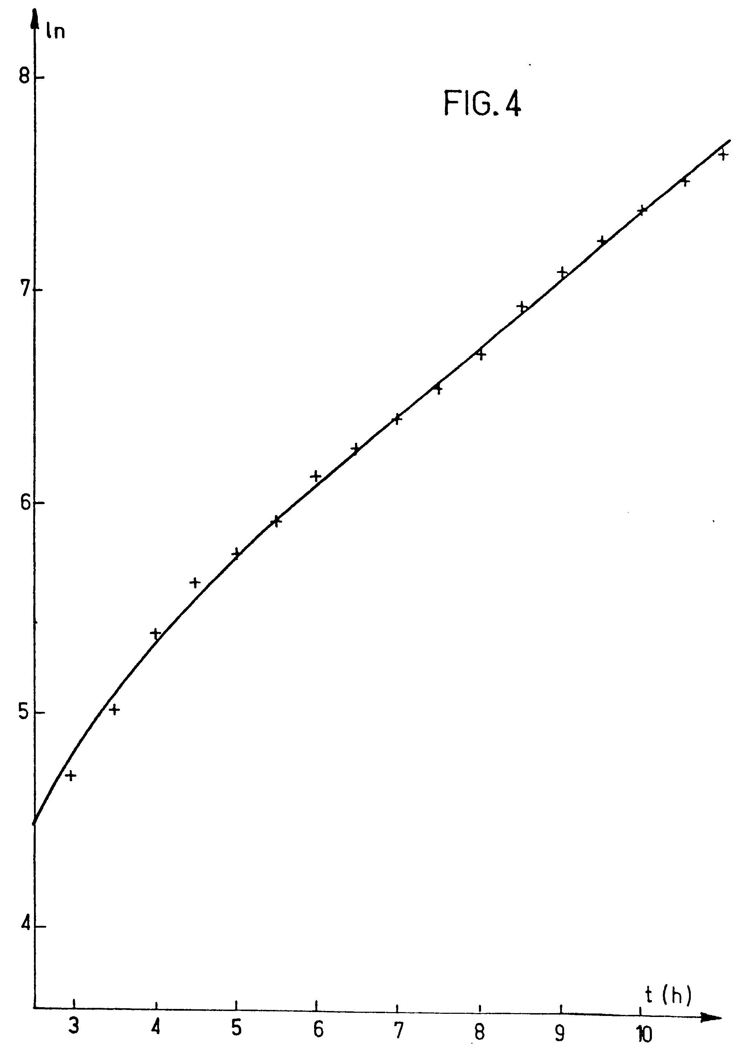

A la figure 4, concernant la souche NCDO 1119, on note un taux de croissance élevé de l'ordre de 0,70 pendant les 4 premières heures. A la sixième heure, le taux de lactate a atteint 28 g/l, à partir de ce moment, on a modifié l'alimentation en eau, en l'accroissant de 66%, ce qui a eu pour effet de réduire la concentration en ammoniaque assurant ainsi une introduction d'ammoniaque dans le fermenteur apte à maintenir cette concentration de 28 g/l qui correspond à un taux de croissance de 0,32 de la 6ème heure à la 11ème heure. On note que l'ultrafiltration est mise en oeuvre vers 7 heures. En fin de fermentation, la concentration bactérienne est de 26 g de bactéries sèches par kg de culture. Le rendement en substrats est de 9,2 kg de lactose par kg de bactéries sèches.

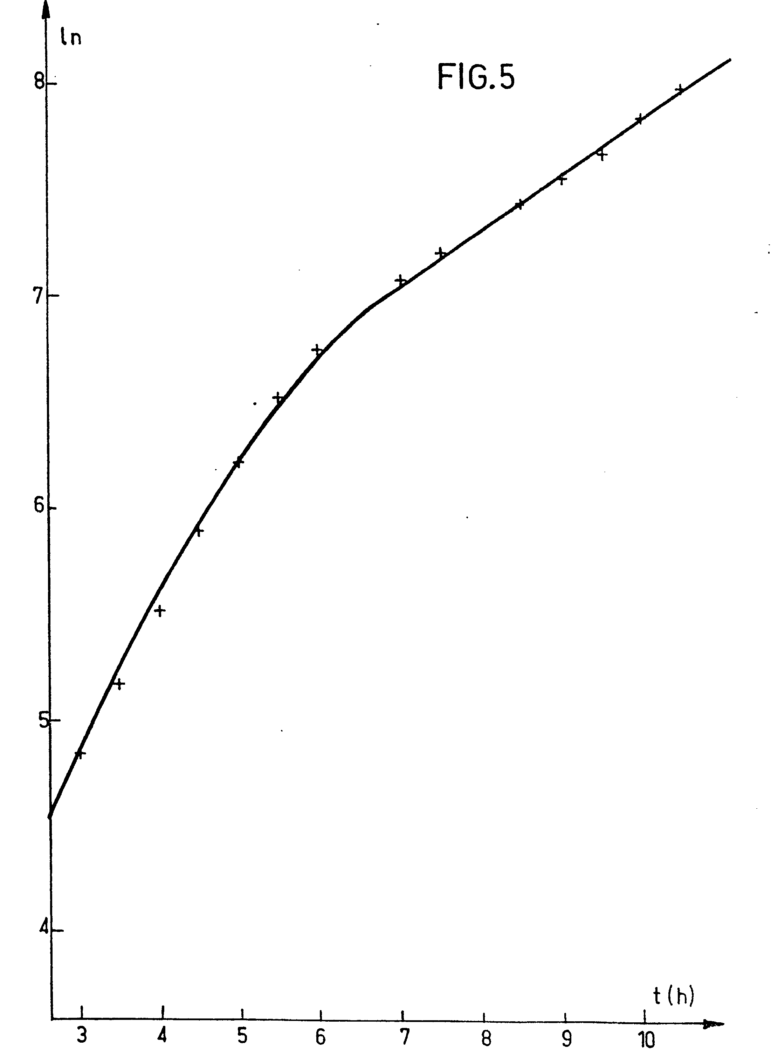

A la figure 5, concernant la souche CNRZ 125, on note un taux de croissance élevé de l'ordre de 0,80 pendant les 5 premières heures. Un peu avant la 8ème heure, le taux de lactate a atteint 32 g/l, et à partir de ce moment, on a modifié l'alimentation en eau, en l'accroissant d'environ 42%, ce qui a eu pour effet de réduire la concentration en ammoniaque, assurant ainsi une introduction d'amminiaque dans le fermenteur apte à maintenir cette concentration de 32 g/l qui correspond à un taux de croissance de 0,26 de la 8ème heure à la 12ème heure. On note que l'ultrafiltration est mise en oeuvre avant la 7ème heure. En fin de fermentation la concentration bactérienne est de 18 g de bactéries sèches par kg de culture. Le rendement en substrats est de 7,9 kg de lactose par kg de bactéries sèches.

L'invention s'applique à la fabrication de microorganismes et plus particulièrement de bactéries lactiques quelles que soient leur utilisation: industrie fromagère, industrie vinicole, etc.

**Revendications**

1. Procédé de fabrication de microorganismes, du genre où on alimente un milieu de culture, préalablement ensemencé, en substrat nutritif, en agent neutralisant, destiné à maintenir constant le PH du milieu de culture, le tout dilué dans de l'eau et où l'on effectue sur ledit milieu de culture une opération d'ultrafiltration d'élimination partielle d'un agent inhibiteur formé et cela pendant au moins une partie de la phase de développement des dits microorganismes, du genre où l'on assure un taux de croissance des microorganismes dudit milieu de culture qui présente, dans une première phase initiale de développement, une valeur élevée et dans une phase successive une valeur constante modérée, caractérisé par la combinaison des mesures suivantes:

a) pendant toute la durée de la phase de fabrication, le PH est compris entre 5,5 et 7,5;

b) on règle les apports en substrat et en eau par rapport à l'apport en agent neutralisant et cela, par détermination permanente de la concentration en agent inhibiteur résultant de l'action dudit agent neutralisant;

c) l'ensemble de ces apports s'effectue directement dans le fermenteur durant toute la fabrication;

d) le taux de croissance modéré est compris entre 0,10 et 0,50;

e) le début de la phase à taux de croissance modéré est déterminé par l'obtention d'une concentration critique dans le milieu de culture de l'agent inhibiteur qui correspond à une concentration maximale admissible pour ledit taux de croissance modéré choisi;

f) on asservit au moins la phase essentielle de l'opération d'ultrafiltration au maintien d'un volume constant dudit milieu de culture, dans lequel se retrouve la totalité des microorganismes fabriqués.

2. Procédé de fabrication selon la revendication 1, caractérisé en ce que le taux de croissance plus modéré est compris entre 0,15 et 0,45.

3. Procédé de fabrication selon la revendication 2, caractérisé en ce que le taux de croissance plus modéré est compris entre 0,20 et 0,40.

4. Procédé de fabrication selon la revendication 3, caractérisé en ce que le taux de croissance plus modéré est compris entre 0,30 et 0,35.

5. Procédé de fabrication de microorganismes selon l'une quelconque des revendications 1 à 5, caractérisé en ce que les débits de substrat et d'eau sont maintenus à des valeurs constantes relativement au débit d'agent neutralisant pendant toute la phase de développement initial à taux de croissance élevé et, dans d'autres rapports, pendant toute la phase successive de développement à taux de croissance plus modéré, pendant laquelle le débit relatif d'eau est rendu plus élevé, d'autant plus élevé que la concentration maximale admissible en agent inhibiteur est choisie plus faible.

6. Procédé de fabrication selon la revendication 1, caractérisé en ce que l'on met en oeuvre, avant l'opération d'ultrafiltration, une phase préliminaire de développement du milieu de culture à volume croissant à partir d'un initial et cela, jusqu'à atteindre le volume maximal, après quoi on met en oeuvre l'opération d'ultrafiltration.

7. Procédé de fabrication selon la revendication 1, caractérisé en ce que la phase d'ultrafiltration provoque d'abord une réduction du volume du milieu de culture, puis un maintien à une valeur moyènne constante dudit volume du milieu de culture ainsi réduit.

8. Procédé de fabrication selon la revendication 7, caractérisé en ce que l'opération d'ultrafiltration, une fois terminée, on poursuit l'alimentation du milieu de culture jusqu'à un niveau pouvant correspondre au volume maximal.

9. Procédé de fabrication de bactéries lactiques, du genre où l'on alimente un milieu de culture, préalablement ensemensé, en substrat nutritif comportant avantageusement du lactose et de la levure, et en ammoniaque ou soude formant agent neutralisant pour maintenir le PH à une valeur constante, le tout dilué dans de l'eau et arrivant directement dans le fermenteur, où l'on effectue sur ledit milieu de culture une opération d'ultrafiltration d'élimination partielle de lactate d'ammonium (ou sodium) et cela pendant au moins une partie de la phase de développement des dites bactéries lactiques, du genre où l'on assure un taux de croissance des bactéries dudit milieu de culture qui présente, dans une première phase initiale de développement, une valeur élevée et dans une phase successive une valeur constante plus modérée, caractérisé par la combinaison des mesures suivantes:

a) le PH du milieu de culture est maintenu à une valeur comprise entre 6 et 7;

b) on règle les apports en substrat et en eau par rapport à l'apport préréglé selon a) en ammoniaque (ou soude) et cela par détermination permenente de la concentration en lactate d'ammonium (ou sodium) résultant de l'action de l'ammonique (ou soude) sur l'acide lactique formé lors de la dégradation du lactose.

c) le début de la phase à taux de croissance plus modéré est déterminé par l'obtention d'une concentration critique, dans le milieu de culture, du lactate d'ammonium (ou sodium), la concentration critique en lactate d'ammonium étant comprise entre 19 g/l pour un taux de croissance de 0,50 et 54 g/l pour un taux de croissance de 0,10.

d) on asservit au moins la phase essentielle de l'opération d'ultrafiltration au maintien d'un volume constant dudit milieu de culture dans lequel se retrouve la totalité des microorganismes fabriques.

10. Procédè de fabrication selon la revendication 9, caractérisé en ce que pendant la phase de développement à taux de croissance plus modéré, la concentration dans le milieu de culture en lactose est comprise entre 50 g/l (pour un taux de croissance de 0,10) et 18 g/l (pour un taux de croissance de 0,50), la concentration en extrait de levure est comprise entre 8 g/l (pour un taux de croissance de 0,10) et 18 g/l (pour un taux de croissance de 0,50), la concentration en ammoniaque étant comprise alors entre 0,50 N (pour un taux de croissance de 0,10) et 0,18 N (pour un taux de croissance de 0,50).

**Patentansprüche**

1. Verfahren zur Herstellung von Mikroorganismen, wobei man in ein vorher beimpftes Kulturmedium Nährsubstrat und ein Neutralisiermittel, das dazu bestimmt ist, den pH-Wert des Kulturmediums konstant zu halten, das Ganze mit Wasser verdünnt, einführt und wobei man mit dem Kulturmedium eine Ultrafiltration zur Teilentfernung eines gebildeten Inhibitors während wenigstens eines Teils der Entwicklungsphase dieser Mikroorganismen derart durchführt, daß man einen Wachstumsgrad der Mikroorganismen dieses Kulturmediums gewährleistet, der in der ersten Anfangsphase der Entwicklung einen erhöhten Wert und in einer anschließenden Phase einen geringeren konstanten Wert hat, gekennzeichnet durch die Kombination der folgenden Maßnahmen:

a) Während der gesamten Dauer der Herstellungsphase liegt der PH-Wert zwischen 5,5 und 7,5.

b) Man steuert die Zugabe an Substrat und an Wasser in bezug auf die Zugabe an Neutralisiermittel durch ständige Bestimmung der aus der Wirkung dieses Neutralisiermittels stammenden Inhibitorkonzentration.

c) Die Gesamtheit dieser Zugaben erfolgt direkt in dem Fermentiergerät während der gesamten Herstellung.

d) Der geringere Wachstumsgrad liegt zwischen 0,10 und 0,50.

e) Der Anfang der Phase mit geringerem Wachstumsgrad wird bestimmt, indem man in dem Kulturmedium eine kritische Inhibitorkonzentration erhält, die einer für den ausgewählten niedrigeren Wachstumsgrad maximal zulässigen Konzentration entspricht.

f) Man regelt wenigstens die wesentliche Phase der Ultrafiltration, indem man ein konstantes Volumen dieses Kulturmediums, in welchem sich die Gesamtheit der hergestellten Mikroorganismen befindet, aufrechterhält.

2. Verfahren zur Herstellung nach Anspruch 1, dadurch gekennzeichnet, daß der geringere Wachstumsgrad zwischen 0,15 und 0,45 liegt.

3. Verfahren zur Herstellung nach Anspruch 2, dadurch gekennzeichnet, daß der geringere Wachstumsgrad zwischen 0,20 und 0,40 liegt.

4. Verfahren zur Herstellung nach Anspruch 3, dadurch gekennzeichnet, daß der geringere Wachstumsgrad zwischen 0,30 und 0,35 liegt.

5. Verfahren zur Herstellung von Mikroorganismen nach einem der Ansprüche 1 bis 4, dadurch gekennzeichnet, daß die Mengen an Substrat und Wasser während der gesamten anfänglichen Entwicklungsphase mit erhöhtem Wachstumsgrad und in anderen Beziehungen während der gesamten anschließenden Entwicklungsphase mit geringerem Wachstumsgrad, während welcher die relative Wassermenge so viel erhöht wird, wie die maximal zulässige Inhibitorkonzentration niedriger ausgewählt wird, auf konstanten Werten bezüglich der Neutralisiermittelmenge gehalten werden.

6. Verfahren zur Herstellung nach Anspruch 1, dadurch gekennzeichnet, daß man vor der Ultrafiltration eine einleitende Entwicklungsphase

des Kulturmediums mit steigendem Volumen, ausgehend von einem Ausgangsvolumen bis zum Erreichen des Maximalvolumens, nach welchem man die Ultrafiltration beginnt, durchführt.

7. Verfahren zur Herstellung nach Anspruch 1, dadurch gekennzeichnet, daß die Ultrafiltrationsphase zuerst eine Volumenminderung des Kulturmediums und dann eine Aufrechterhaltung dieses so verminderten Volumens des Kulturmediums auf einem konstanten mittleren Wert hervorruft.

8. Verfahren zur Herstellung nach Anspruch 7, dadurch gekennzeichnet, daß man nach Beendigung der Ultrafiltration die Einspeisung in das Kulturmedium bis zu einem Niveau, das einem Maximalvolumen entsprechen kann, fortsetzt.

9. Verfahren zur Herstellung von Milchbakterien, wobei man in ein vorher beimpftes Kulturmedium Nährsubstrat, das vorteilhafterweise Lactose und Hefe enthält, sowie Neutralisiermittel bildendes Ammoniak oder Soda, um den pH-Wert auf einem konstanten Wert zu halten, das Ganze mit Wasser verdünnt, direkt in das Fermentiergerät einführt und wobei man mit diesem Kulturmedium eine Ultrafiltration zur Teilentfernung von Ammoniumlactat (oder Natriumlactat) während wenigstens eines Teils der Entwicklungsphase dieser Milchbakterien derart durchführt, daß man einen Wachstumsgrad der Bakterien dieses Kulturmediums gewährleistet, welcher in einer ersten Anfangsphase der Entwicklung einen erhöhten Wert und während einer anschließenden Phase einen konstanten geringeren Wert besitzt, gekennzeichnet durch die Kombination folgender Maßnahmen:

a) Der pH-Wert des Kulturmediums wird auf einem Wert zwischen 6 und 7 gehalten.

b) Man steuert die Zugaben an Substrat und an Wasser in Abhängigkeit von der vorher gemäß a) geregelten Zugabe an Ammoniak (oder Soda) durch ständige Bestimmung der Ammoniumlactat- (oder Natriumlactat)-Konzentration, die aus der Einwirkung des Ammoniak (oder Soda) auf die während des Lactoseabbaus gebildeten Milchsäure stammt.

c) Der Anfang der Phase mit niedrigerem Wachstumsgrad wird bestimmt, indem man in dem Kulturmedium eine kritische Ammoniumlactat- (oder Natriumlactat)-Konzentration erhält, wobei die kritische Ammoniumlactatkonzentration zwischen 19 g/l für einen Wachstumsgrad von 0,50 und 54 g/l für einen Wachstumsgrad von 0,10 liegt.

d) Man steuert wenigstens die wesentliche Phase der Ultrafiltration so, daß man ein konstantes Volumen dieses Kulturmediums, in welchem sich die Gesamtheit der hergestellten Mikroorganismen befinden, aufrechterhält.

10. Verfahren zur Herstellung nach Anspruch 9, dadurch gekennzeichnet, daß während der Entwicklungsphase mit niedrigerem Wachstumsgrad in dem Kulturmedium die Lactosekonzentration zwischen 50 g/l (für einen Wachstumsgrad von 0,10) und 18 g/l (für einen Wachstumsgrad von 0,50) liegt, die Konzentration an Hefeextrakt zwischen 8 g/l (für einen Wachstumsgrad von 0,10) und 18 g/l (für einen Wachstumsgrad von 0,50) liegt und die Ammoniumkonzentration dann zwischen 0,50 N (für einen Wachstumsgrad von 0,10) und 0,18 N (für einen Wachstumsgrad von 0,50) liegt.

## Claims

1. Process for the production of microorganisms by feeding a previously inoculated culture medium with a nutritive substrate, a neutrilizing agent for maintaining the pH of the culture medium constantly, diluted by water, and by effecting an operation of ultrafiltration with said culture medium for a partial elimination of a formed inhibitor agent during at least a part of the phase of development of the said microorganisms so that a degree of growth of the microorganisms of said culture medium is assured which presents in a first initial phase of development an elevated value and in a succeeding phase a constant moderate value, characterized by the combination of the following measures:

a) During the entire duration of the phase of production the pH is between 5.5 and 7.5.

b) The additions of substrate and water are adjusted in relation to the addition of the neutralizing agent by permanent determination of the concentration of inhibitor agent resulting from the action of said neutralizing agent.

c) The totality of the additions is effected directly in the fermenter during the entire production.

d) The moderate degree of growth is between 0.10 and 0.50.

e) The start of the phase of moderate degree of growth is determined by obtaining in the culture medium a critical concentration of the inhibitor agent which corresponds to a maximum concentration admissible for said chosen moderate degree of growth.

f) At least the essential phase of the operation of ultrafiltration is controlled maintaining a constant volume of said culture medium in which the totality of the produced microorganisms is present.

2. Process of production according to claim 1, characterized in that the degree of growth more moderate is between 0.15 and 0.45.

3. Process of production according to claim 2, characterized in that the degree of growth more moderate is between 0.20 and 0.40.

4. Process of production according to claim 3, characterized in that the degree of growth more moderate is between 0.30 and 0.35.

5. Process for the production of microorganisms according to one of the claims 1 to 4, characterized in that the amounts of substrate and water are maintained at constant values in relation to the amount of neutralizing agent during the entire phase of initial development at elevated degree of growth and in other relations during the entire succeeding phase of development at a degree of growth more moderated during which the relative amount of water is elevated as the admissible maximum concentration of inhibitor agent is chosen lower.

6. Process of production according to claim 1, characterized in that before the operation of ultrafiltration a preliminary phase of development of the culture medium with a volume increasing from an initial volume is carried out until the maximum volume is reached after which the operation of ultrafiltration is carried out.

7. Process of production according to claim 1, characterized in that the phase of ultrafiltration causes first a reduction of volume of the culture medium, then maintaining a medium constant value of said volume of the culture medium so reduced.

8. Process of production according to claim 7, characterized in that when the operation of ultrafiltration is terminated feeding of the culture medium is prosecuted until a level which may correspond to the maximum volume.

9. Process for the production of lactic bacteria by feeding a previously inoculated culture medium with a nutritive substrate comprising advantageously lactose and yeast and ammonia or soda forming a neutralizing agent to maintain the pH at a constant value, diluted by water and arriving directly in the fermenter where with said culture medium an operation of ultrafiltration of partial elimination of ammonium (or sodium) lactate during at least a part of the phase of development of said lactic bacteria is carried out, assuring a degree of growth of the bacteria of said culture medium which presents in a first initial phase of development an elevated value and in a succeeding phase a more moderate constant value, characterized by the combination of the following measures:

a) The pH of the culture medium is maintained at a value between 6 and 7.

b) The additions of substrate and water are regulated in relation to the addition of ammonia (or soda) preregulated according to a) by permanent determination of the concentration of ammonium (or sodium) lactate resulting from the action of ammonia (or soda) on the lactic acid formed during the degradation of lactose.

c) The start of the phase with a more moderate degree of growth is determined by obtaining in the culture medium a critical concentration of ammonium (or sodium) lactate, the critical concentration of ammonium lactate being between 19 g/l for a degree of growth of 0.50 and 54 g/l for a degree of growth of 0.10.

d) At least the essential phase of the operation of ultrafiltration is controlled to maintain a constant volume of said culture medium in which the totality of the produced microorganisms is present.

10. Process of production according to claim 9, characterized in that during the phase of development at a more moderate degree of growth the concentration of lactose in the culture medium is between 50 g/l (for a degree of growth of o.10) and 18 g/l (for a degree of growth of 0.50), the concentration of extract of yeast is between 8 g/l (for a degree of growth of 0.10) and 18 g/l (for a degree of growth of 0.50), the concentration of ammonia being then between 0.50 N (for a degree of growth of 0.10 N (for a degree of growth of 0.50).

FIG.1

0 065 895

FIG.2

FIG.3

FIG. 4

FIG.5